# EUROPEAN PATENT APPLICATION

(11) **EP 4 484 923 A1**
(43) Date of publication of application: **01.01.2025**
(21) Application number: 23779646.1
(22) Date of filing: 15.03.2023
(51) Int. Cl.: G01N 21/05, C12M 1/00, C12Q 1/02, G01N 21/65

(54) **FLOW CELL, AND MEASURING METHOD**

(30) Priority: 30.03.2022 JP 2022057535; 13.03.2023 JP 2023039114
(71) Applicant: FUJIFILM Corporation, Tokyo 106-8620 (JP)
(72) Inventor: NISHI, Takayuki, Ashigarakami-gun, Kanagawa 258-8577 (JP); YAMAMOTO, Takashi, Ashigarakami-gun, Kanagawa 258-8577 (JP); HASEGAWA, Masataka, Ashigarakami-gun, Kanagawa 258-8577 (JP); KOBAYASHI, Yuka, Ashigarakami-gun, Kanagawa 258-8577 (JP)
(74) Representative: Hoffmann Eitle
(86) International application number: PCT/JP2023/010196
(87) International publication number: WO 2023/189628

(57) **Abstract**

A flow cell includes a main body that includes a flow passage through which fluid containing a substance from which physical property data is to be measured flows, and an optical system which is disposed on a part of a wall surface forming the flow passage and condenses measurement light for the physical property data and of which an emission surface for the measurement light in contact with the fluid is a flat surface.

## Description

### BACKGROUND OF THE INVENTION

### 1. Field of the Invention

A technology of the present disclosure relates to a flow cell and a measuring method.

### 2. Description of the Related Art

A flow cell that includes a flow passage through which fluid flows and is used to measure physical property data of a substance present in the fluid is known. Some flow cells include an optical system that condenses measurement light for physical property data and takes in light returning from a substance irradiated with the measurement light. The fluid is, for example, a cell culture solution containing a cell product, such as an antibody, as the substance, and the physical property data is, for example, Raman spectral data.

JP2020-511635A discloses a flow cell that includes a ball lens as an optical system. The ball lens is disposed in an orifice provided in a wall surface forming a flow passage. An emission surface of the ball lens for measurement light is in contact with fluid flowing through the flow passage.

### SUMMARY OF THE INVENTION

In JP2020-511635A, the ball lens having an optical power on an emission surface is used as an optical system as described above. For this reason, a condensing position of the measurement light is away from the emission surface of the ball lens in contact with the fluid. That is, the fluid is interposed between the emission surface of the ball lens and the condensing position of the measurement light. Therefore, there is a concern that the amount of light required for obtaining physical property data capable of withstanding analysis may not be obtained since the measurement light is attenuated by the fluid. This problem is more significant in a case of fluid having a relatively high turbidity, such as a cell culture solution.

One embodiment according to the technology of the present disclosure provides a flow cell and a measuring method capable of reducing a concern that the amount of measurement light required for obtaining physical property data capable of withstanding analysis may not be obtained.

A flow cell according to an aspect of the present disclosure comprises a main body that includes a flow passage through which fluid containing a substance from which physical property data is to be measured flows, and an optical system which is disposed on a part of a wall surface forming the flow passage and condenses measurement light for the physical property data and of which an emission surface for the measurement light in contact with the fluid is a flat surface.

It is preferable that the optical system includes a lens having a positive optical power, and an optical element of which an emission surface for the measurement light in contact with the fluid is a flat surface.

It is preferable that the lens is any one of a ball lens, a hemispherical lens, or a cylindrical lens.

In a case where the lens is the cylindrical lens, it is preferable that the cylindrical lens condenses the measurement light in a form of a line parallel or perpendicular to a direction in which the fluid flows.

It is preferable that the optical element is a transparent plate of which an emission surface for the measurement light in contact with the fluid and an incident surface for the measurement light opposite to the emission surface for the measurement light in contact with the fluid are parallel to each other.

It is preferable that a thickness of the optical element in a direction of an optical axis is equal to or smaller than a distance between a point at which an emission surface of the lens and the optical axis intersect with each other and a condensing position of the measurement light.

It is preferable that the thickness is equal to or greater than a half of the distance.

It is preferable that the thickness is equal to the distance and the condensing position is positioned on the emission surface of the optical element.

It is preferable that the thickness is 0.3 mm or more and 7.5 mm or less.

It is preferable that a curvature of an emission surface of the lens and a curvature of an incident surface of the optical element are equal to each other. Further, it is preferable that the emission surface of the lens and the incident surface of the optical element are bonded to each other.

It is preferable that a connector, to which an optical analysis apparatus outputting the physical property data is connected and in which the lens is disposed at a distal end, is attachably and detachably provided on the main body.

It is preferable that the optical system includes a lens which has a positive optical power and of which an emission surface for the measurement light in contact with the fluid is a flat surface.

It is preferable that the lens is any one of a hemispherical lens or a cylindrical lens.

In a case where the lens is the cylindrical lens, it is preferable that the cylindrical lens condenses the measurement light in a form of a line parallel or perpendicular to a direction in which the fluid flows.

It is preferable that the wall surface is a smooth surface.

It is preferable that a turbidity of the fluid is 250 NTU or more and 1000 NTU or less.

It is preferable that the physical property data is Raman spectral data.

A measuring method according to another aspect of the present disclosure measures the physical property data using the flow cell described above.

It is preferable that the fluid is a cell culture solution. In this case, it is preferable that the cell culture solution contains a cell product as the substance.

It is preferable that the cell culture solution is obtained from a culture vessel in which culture is being performed. Further, it is preferable that the cell culture solution is a solution from which cells have been removed.

According to the technology of the present disclosure, it is possible to provide a flow cell and a measuring method capable of reducing a concern that the amount of measurement light required for obtaining physical property data capable of withstanding analysis may not be obtained.

### BRIEF DESCRIPTION OF THE DRAWINGS

Fig. 1 is a diagram showing an aspect where Raman spectral data of a substance present in a cell culture solution obtained from a culture vessel in which culture is being performed is measured.
Fig. 2 is a perspective view of a flow cell.
Fig. 3 is an exploded cross-sectional view of the flow cell.
Fig. 4 is an exploded perspective view of a transparent plate, an O-ring, and a plate presser.
Fig. 5 is a cross-sectional view of the flow cell.
Fig. 6 is a cross-sectional view of the flow cell.
Fig. 7 is a diagram showing another example of a positional relationship between a ball lens and the transparent plate.
Fig. 8 is a diagram showing another example of an optical element.
Fig. 9 is a diagram showing an optical system that includes a hemispherical lens and a transparent plate.
Fig. 10 is a diagram showing an optical system that includes a plano-convex lens and a transparent plate.
Fig. 11 is a diagram showing an optical system that includes a plano-convex aspheric lens and a transparent plate.
Fig. 12 is a diagram showing an optical system that includes a cylindrical lens and a transparent plate.
Fig. 13 is a diagram showing an example in which excitation light is condensed in the form of a line parallel to a direction in which culture supernatant liquid flows by the cylindrical lens.
Fig. 14 is a diagram showing an example in which excitation light is condensed in the form of a line perpendicular to a direction in which culture supernatant liquid flows by the cylindrical lens.
Fig. 15 is a diagram showing an example in which only the hemispherical lens is used.
Fig. 16 is a diagram showing an example in which excitation light is condensed in the form of a line parallel to a direction in which culture supernatant liquid flows by the cylindrical lens.
Fig. 17 is a diagram showing an example in which excitation light is condensed in the form of a line perpendicular to a direction in which culture supernatant liquid flows by the cylindrical lens.
Fig. 18 is a diagram showing an optical system that is formed of only the plano-convex lens shown in Fig. 10.
Fig. 19 is a diagram showing an optical system that is formed of only the plano-convex lens shown in Fig. 11.
Fig. 20 is a diagram showing an optical system in which the hemispherical lens and the transparent plate shown in Fig. 9 are integrally formed as one lens.
Fig. 21 is a table in which items of conditions and evaluation in Examples and Comparative examples are summarized.

### DESCRIPTION OF THE PREFERRED EMBODIMENTS

### [First embodiment]

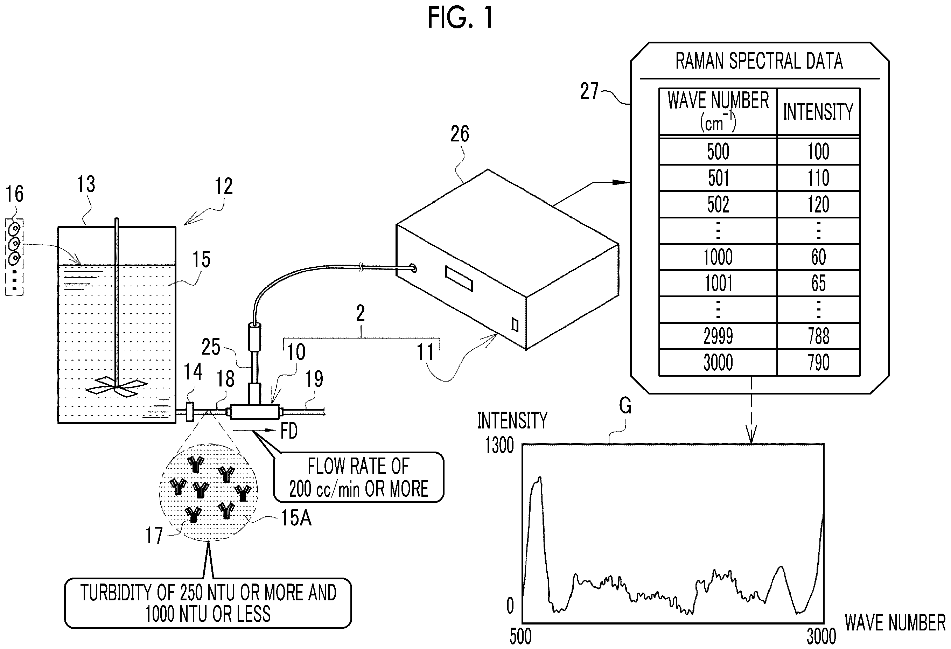

For example, a measurement system 2 comprises a flow cell 10 and a Raman spectrometer 11 as shown in Fig. 1. The measurement system 2 is incorporated into, for example, a cell culture unit 12 of a system for manufacturing a drug substance of a biopharmaceutical. The cell culture unit 12 includes a culture vessel 13 and a cell-removing filter 14. A cell culture solution 15 is stored in the culture vessel 13.

An antibody-producing cell 16 is seeded in the culture vessel 13, and is cultured in the cell culture solution 15. The antibody-producing cell 16 is, for example, a cell established by incorporating an antibody gene into a host cell such as Chinese hamster ovary (CHO) cells. The antibody-producing cell 16 produces immunoglobulin, that is, an antibody 17 in a culture process. For this reason, not only the antibody-producing cell 16 but also the antibody 17 is present in the cell culture solution 15. The antibody 17 is, for example, a monoclonal antibody, and is an active ingredient of a biopharmaceutical. The antibody 17 is an example of "a substance" and "a cell product" according to a technology of the present disclosure.

A first sending passage 18 is connected to the culture vessel 13. The cell-removing filter 14 is disposed in the first sending passage 18. The cell-removing filter 14 captures the antibody-producing cell 16 contained in the cell culture solution 15 with a filter membrane (not shown) using, for example, a tangential flow filtration (TFF) method and removes the antibody-producing cell 16 from the cell culture solution 15. Further, the cell-removing filter 14 transmits the antibody 17. For this reason, the cell culture solution 15 mainly containing the antibody 17 flows downstream of the cell-removing filter 14 of the first sending passage 18. The cell culture solution 15 from which the antibody-producing cell 16 has been removed by the cell-removing filter 14 in this way is called culture supernatant liquid. Hereinafter, the cell culture solution 15 from which the antibody-producing cell 16 has been removed by the cell-removing filter 14 will be referred to as culture supernatant liquid 15A. The culture supernatant liquid 15A is an example of "fluid" according to the technology of the present disclosure.

A turbidity of the culture supernatant liquid 15A is 250 nephelometric turbidity units (NTU) or more and 1000 NTU or less. NTU is a unit of a turbidity of liquid based on a formazin standard solution.

The culture supernatant liquid 15A also contains impurities, such as cell-derived protein, cell-derived deoxyribonucleic acid (DNA), and an aggregate of the antibody 17, or viruses and the like, in addition to the antibody 17. The impurities are also an example of "a substance" and "a cell product" according to the technology of the present disclosure. Further, viruses and the like are also an example of "a substance" according to the technology of the present disclosure.

The flow cell 10 is connected to the first sending passage 18 on the downstream side of the cell-removing filter 14. The culture supernatant liquid 15A from the first sending passage 18 flows through the flow cell 10 as shown by an arrow FD. A sending pump (not shown) is provided downstream of the cell-removing filter 14 of the first sending passage 18 (between the cell-removing filter 14 and the flow cell 10). The sending pump sends the culture supernatant liquid 15A toward the flow cell 10 at a flow rate of 200 cc/min or more, for example, 300 cc/min.

A second sending passage 19 is also connected to the flow cell 10. The culture supernatant liquid 15A that has flowed into the flow cell 10 from the first sending passage 18 flows out to the second sending passage 19. The second sending passage 19 is connected to, for example, a purification unit that purifies the antibody 17 from the culture supernatant liquid 15A using a chromatography device, and sends the culture supernatant liquid 15A from the flow cell 10 to the purification unit. The first sending passage 18 may be connected to the purification unit, and a branch passage may be provided downstream of the cell-removing filter 14 of the first sending passage 18. Then, the flow cell 10 may be connected to the branch passage, and the culture supernatant liquid 15A that has flowed through the flow cell 10 may be disposed of. Alternatively, the second sending passage 19 may be connected to the culture vessel 13, and the culture supernatant liquid 15A that has flowed through the flow cell 10 may be returned to the culture vessel 13.

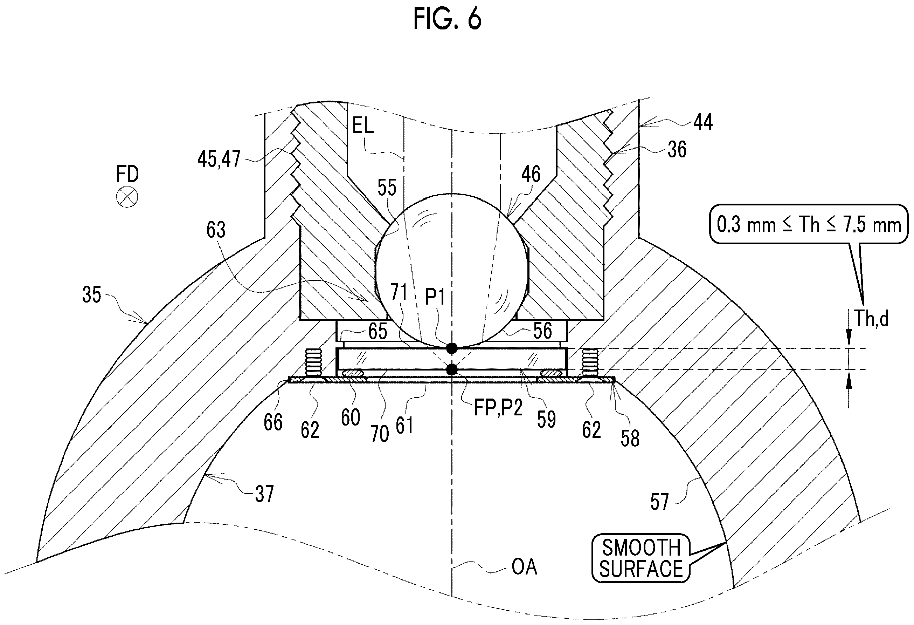

The Raman spectrometer 11 is an apparatus that evaluates a substance using characteristics of Raman scattered light, and is an example of "an optical analysis apparatus" according to the technology of the present disclosure. In a case where a substance is irradiated with excitation light EL (see Fig. 6), Raman scattered light having a wavelength different from that of the excitation light EL is generated due to an interaction between the excitation light EL and the substance. A difference in wavelength between the excitation light EL and the Raman scattered light corresponds to the energy of molecular vibration of the substance. For this reason, it is possible to obtain Raman scattered light having different wave numbers between substances having different molecular structures. The excitation light EL is an example of "measurement light" according to the technology of the present disclosure. Of a Stokes line and an anti-Stokes line, it is preferable to use the Stokes line as the Raman scattered light.

The Raman spectrometer 11 includes a sensor unit 25 and an analyzer 26. A distal end of the sensor unit 25 is connected to the flow cell 10. The sensor unit 25 emits the excitation light EL from an emission port provided at the distal end thereof. The culture supernatant liquid 15A that flows in the flow cell 10 is irradiated with the excitation light EL. Raman scattered light is generated due to an interaction between the excitation light EL and the antibody 17 or the like contained in the culture supernatant liquid 15A. The sensor unit 25 receives the Raman scattered light and outputs the received Raman scattered light to the analyzer 26. In the present embodiment, laser light was used as the excitation light EL, an output of the laser light was set to 500 mW, a central wavelength thereof was set to 785 nm, an irradiation time thereof was set to 1 second, and the number of times of integration thereof was set to 10. Further, a diameter of the laser light was set to 3.50 mm.

The analyzer 26 decomposes the Raman scattered light for each wave number and derives an intensity of the Raman scattered light for each wave number to generate Raman spectral data 27. The Raman spectral data 27 is an example of "physical property data" according to the technology of the present disclosure. The analyzer 26 is connected to an information processing apparatus (not shown) through a computer network, such as a local area network (LAN), to be capable of communicating with the information processing apparatus. The analyzer 26 transmits the generated Raman spectral data 27 to the information processing apparatus. The information processing apparatus is, for example, a personal computer. The information processing device derives the concentration or the compositional ratio of the antibody 17 or the like contained in the culture supernatant liquid 15A on the basis of the Raman spectral data 27 transmitted from the analyzer 26, and displays a result thereof on a display.

The Raman spectral data 27 is data in which the intensity of the Raman scattered light for each wave number is registered. In Fig. 1, the Raman spectral data 27 is data in which an intensity of scattered light in a wave number range of 500 cm⁻¹ to 3000 cm⁻¹ is derived in units of 1 cm⁻¹. A graph G shown below the Raman spectral data 27 is a graph that is obtained in a case where the intensity of the Raman spectral data 27 is plotted for each wave number and the plotted points are connected with a line.

In this way, the measurement system 2 allows the culture supernatant liquid 15A, which is obtained from the culture vessel 13 in which the antibody-producing cell 16 is being cultured, to flow into the flow cell 10. Then, the culture supernatant liquid 15A flowing through the flow cell 10 is irradiated with the excitation light EL through the sensor unit 25, so that the Raman spectral data 27 of the antibody 17 or the like contained in the culture supernatant liquid 15A is measured.

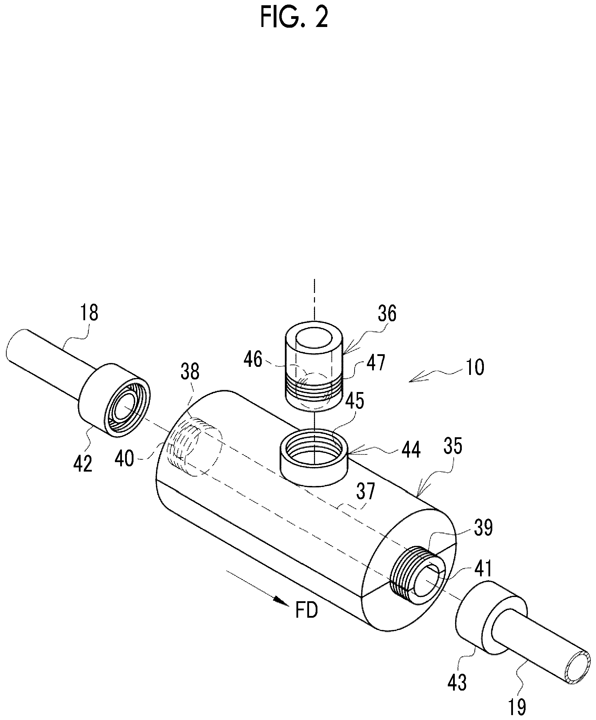

For example, the flow cell 10 includes a main body 35 and a sensor unit connector 36 as shown in Fig. 2. The main body 35 is a cylindrical member that includes a flow passage 37 provided at a center therein and having a linear shape and a circular cross-sectional shape. The main body 35 is made of a metal, for example, Hastelloy, or the like. Alternatively, the main body 35 may be made of a resin, for example, a polyolefin-based resin, or the like.

A first connecting portion 38 and a second connecting portion 39 having the shape of a cylindrical boss are provided at centers of both end surfaces of the main body 35. The first connecting portion 38 includes an inlet 40 of the flow passage 37, and the second connecting portion 39 includes an outlet 41 of the flow passage 37. A direction parallel to the flow passage 37 from the inlet 40 toward the outlet 41 is a direction FD in which the culture supernatant liquid 15A flows. The direction FD is an example of "a direction in which the fluid flows" according to the technology of the present disclosure. Since the flow passage 37 has a circular cross-sectional shape as described above, an outline of the cross-sectional shape of the flow passage 37 as viewed in the direction FD is a curve (see also Fig. 6). In other words, the flow passage 37 as viewed in the direction FD has a shape without corners.

The first connecting portion 38 and the second connecting portion 39 are parallel threads. A sterile connector 42 provided at one end of the first sending passage 18 is liquid-tightly mounted on the first connecting portion 38. Further, a sterile connector 43 provided at one end of the second sending passage 19 is liquid-tightly mounted on the second connecting portion 39. The first connecting portion 38 and the second connecting portion 39 may be taper threads.

A mounting portion 44 is formed in the middle of a peripheral surface of the main body 35. The mounting portion 44 is a hole that is used to attachably and detachably mount the sensor unit connector 36 on the main body 35, and a thread 45 is formed on an inner peripheral surface of the mounting portion 44. The mounting portion 44 is provided up to the flow passage 37, and a distal end portion of the sensor unit connector 36 is housed in the mounting portion 44. Hereinafter, a side on which the mounting portion 44 is formed is referred to as an upper side of the main body 35.

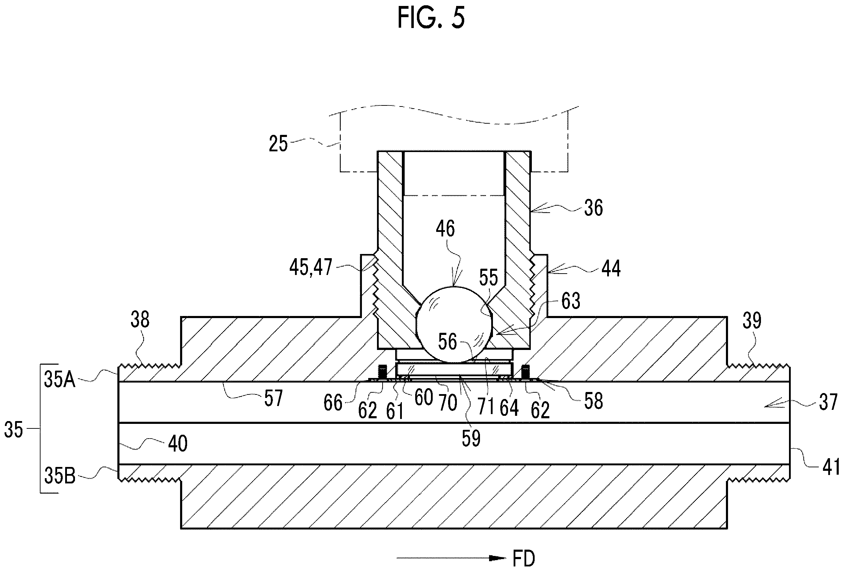

The sensor unit connector 36 is a cylindrical member in which a ball lens 46 is disposed at a distal end. A thread 47 to be screwed into the thread 45 of the mounting portion 44 is formed on an outer peripheral surface of the distal end portion of the sensor unit connector 36. The distal end of the sensor unit 25 is attachably and detachably connected to a proximal end of the sensor unit connector 36 opposite to the distal end (see Fig. 5). The sensor unit connector 36 is an example of "a connector" according to the technology of the present disclosure.

The ball lens 46 is literally a lens having a spherical shape, and is made of, for example, quartz glass. The ball lens 46 condenses the excitation light EL emitted from the sensor unit 25 and takes the Raman scattered light, which is generated due to the interaction between the excitation light EL and the antibody 17 or the like contained in the culture supernatant liquid 15A, into the sensor unit 25. The ball lens 46 is an example of "a lens having a positive optical power" according to the technology of the present disclosure.

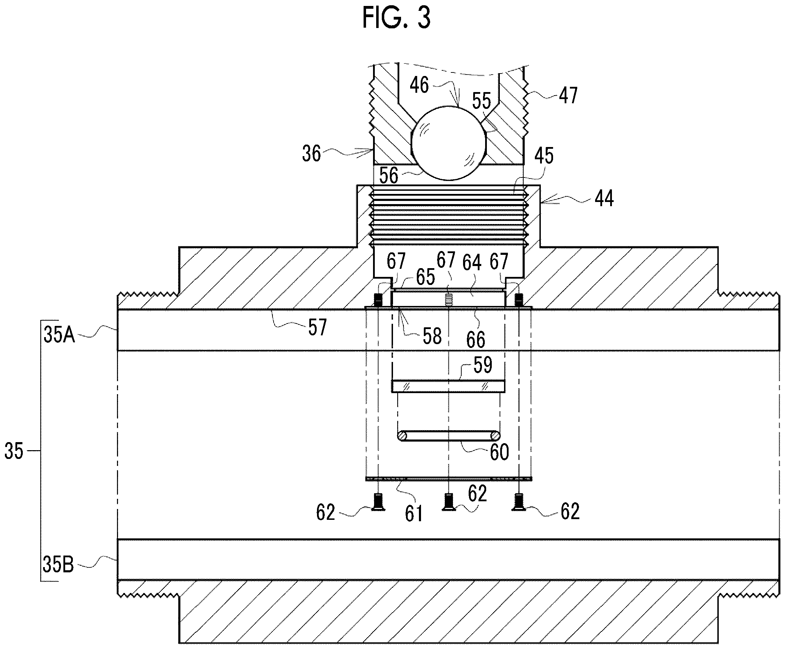

For example, as shown in Fig. 3, the ball lens 46 is fitted into and held by a first holding portion 55 that is formed inside the distal end of the sensor unit connector 36 and has a substantially U-shaped cross section. The ball lens 46 is held by the first holding portion 55 in a state where an emission surface 56 of the ball lens 46 for the excitation light EL is exposed to the outside from the distal end of the sensor unit connector 36.

The main body 35 includes an upper main body 35A and a lower main body 35B. The main body 35 is divided into the upper main body 35A and the lower main body 35B exactly at the center of the flow passage 37. For this reason, in a case where the upper main body 35A and the lower main body 35B are integrated with each other by the adhesion thereof or the like using an adhesive, so that the main body 35 is produced.

In the upper main body 35A, a transparent plate 59, an O-ring 60, and a plate presser 61 are mounted on a boundary portion 58 between the mounting portion 44 and a wall surface 57, which forms the flow passage 37 below the mounting portion 44, by four countersunk screws 62 (only three countersunk screws are shown in Fig. 3). The transparent plate 59 is made of quartz glass having a transmittance of, for example, 95% or more with respect to the excitation light EL and the Raman scattered light, and transmits the excitation light EL emitted from the ball lens 46 and the Raman scattered light emitted from a substance contained in the culture supernatant liquid 15A (see Fig. 6). The transparent plate 59 forms an optical system 63 (see Fig. 5 and the like) together with the ball lens 46. The boundary portion 58 is an example of "a part of the wall surface that forms the flow passage" according to the technology of the present disclosure. The transparent plate 59 is an example of "an optical element" according to the technology of the present disclosure.

The transparent plate 59 is fitted into and held by a second holding portion 64 that is a circular recess consecutively provided below the mounting portion 44. A stopper 65 that is in contact with an edge of the transparent plate 59 and prevents the transparent plate 59 from entering the mounting portion 44 is provided at a boundary between the mounting portion 44 and the second holding portion 64.

The plate presser 61 is fitted into and held by a third holding portion 66 that is a circular recess consecutively provided below the second holding portion 64. The third holding portion 66 is provided with screw holes 67 into which the countersunk screws 62 are screwed. The plate presser 61 may be fixed to the third holding portion 66 by an adhesive instead of the countersunk screws 62.

The O-ring 60 is interposed between the transparent plate 59 and the plate presser 61. The O-ring 60 is rubber having elasticity, and is crushed between the transparent plate 59 and the plate presser 61 in a case where the plate presser 61 is fastened and fixed to the third holding portion 66 by the countersunk screws 62 (see Fig. 5 and the like). The O-ring 60 is crushed between the transparent plate 59 and the plate presser 61 to prevent the culture supernatant liquid 15A flowing through the flow passage 37 from leaking to the mounting portion 44.

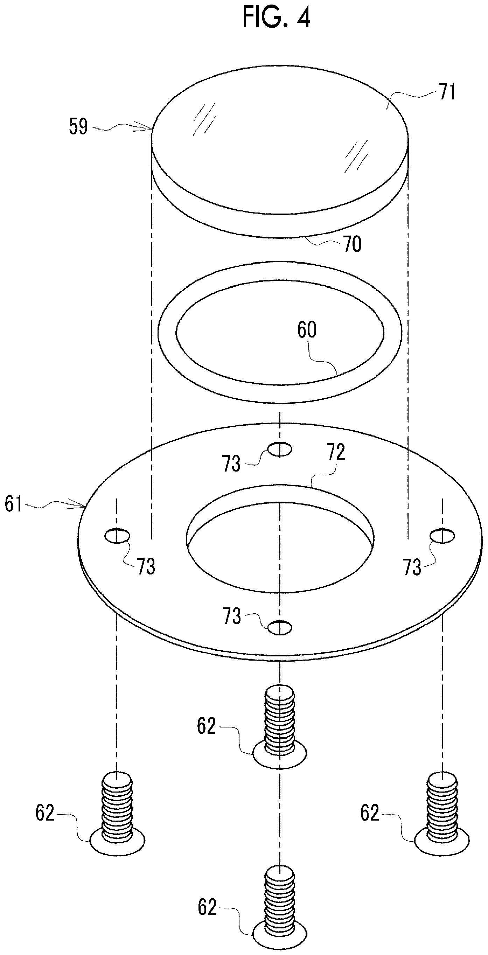

For example, as shown in Fig. 4, the transparent plate 59 is a disk of which an emission surface 70 for the excitation light EL and an incident surface 71 for the excitation light EL opposite to the emission surface 70 are parallel to each other. Here, "parallel" refers to parallel in a meaning including an error that is generally allowed in the technical field to which the technology of the present disclosure belongs, and an error to such an extent not contrary to the spirit and scope of the technology of the present disclosure, in addition to completely parallel.

The O-ring 60 is a circular ring having a diameter slightly smaller than the diameter of the transparent plate 59. The plate presser 61 is an annular thin plate that includes an opening 72 at a central portion thereof. The opening 72 has a diameter slightly smaller than the diameter of the O-ring 60. The opening 72 serves as an emission port for the excitation light EL and an incident port for the Raman scattered light. Insertion holes 73 for the countersunk screws 62 are formed in the plate presser 61 at an interval of 90°.

Figs. 5 and 6 show a state where the transparent plate 59, the O-ring 60, and the plate presser 61 are mounted on the boundary portion 58 by the countersunk screws 62, the upper main body 35A and the lower main body 35B are integrated with each other, and the sensor unit connector 36 is mounted on the mounting portion 44. In this state, the emission surface 56 of the ball lens 46 and the incident surface 71 of the transparent plate 59 are in contact with each other on an optical axis OA (see Fig. 6). The emission surface 70 of the transparent plate 59 is in contact with the culture supernatant liquid 15A that flows through the flow passage 37. The emission surface 70 of the transparent plate 59 is an example of "an emission surface for measurement light in contact with fluid" and "a flat surface" according to the technology of the present disclosure.

In Fig. 6, the excitation light EL emitted from the emission surface 56 of the ball lens 46 is condensed at a condensing position FP. The condensing position FP is determined depending on a diameter, a refractive index, a focal length, and the like of the ball lens 46. The condensing position FP in this case has a dot shape.

In a case where a thickness of the transparent plate 59 in a direction of the optical axis OA is denoted by Th and a distance between a first point P1 at which the emission surface 56 of the ball lens 46 and the optical axis OA intersect with each other and the condensing position FP is denoted by d, the thickness Th is equal to the distance d, that is, "Th = d" is satisfied. In this case, the condensing position FP coincides with a second point P2 at which the emission surface 70 of the transparent plate 59 and the optical axis OA intersect with each other. That is, the condensing position FP is positioned on the emission surface 70 of the transparent plate 59. The thickness Th (and the distance d) is 0.3 mm or more and 7.5 mm or less (0.3 mm ≤ Th ≤ 7.5 mm). The thickness Th (and the distance d) is, for example, 2 mm. "Equal" between the thickness Th and the distance d refers to "equal" in a meaning including an error that is generally allowed in the technical field to which the technology of the present disclosure belongs and an error to such an extent not contrary to the spirit and scope of the technology of the present disclosure, in addition to completely equal (an error of 0). The error referred to herein is preferably ± 10% and more preferably ± 5%.

The wall surface 57 forming the flow passage 37 is a smooth surface. The smooth surface means, for example, a surface having no unevenness of 1 mm or more.

The diameter of the flow passage 37 is in a range in which a Reynolds number Re in the flow passage 37 is 2300 or more (Re ≥ 2300) under conditions where the flow rate of the culture supernatant liquid 15A flowing through the flow passage 37 is 200 cc/min or more as shown in Fig. 1 and the viscosity of the culture supernatant liquid 15A is 0.001 Pa s, which is the same as that of water. In a case where the Reynolds number Re is 2300 or more, turbulence occurs in the culture supernatant liquid 15A that flows through the flow passage 37. Accordingly, a component deviation in the culture supernatant liquid 15A is reduced, so that the measurement stability of the Raman spectral data 27 can be improved. The diameter of the flow passage 37 at the boundary portion 58 where the transparent plate 59 and the like are provided is a distance between the second point P2 at which the emission surface 70 of the transparent plate 59 and the optical axis OA intersect with each other and a point at which the wall surface 57 and the optical axis OA intersect with each other.

Next, the action obtained from the above-mentioned configuration will be described. The measurement system 2 consisting of the flow cell 10 and the Raman spectrometer 11 is incorporated into the cell culture unit 12. The first connecting portion 38 of the flow cell 10 is connected to the first sending passage 18 and the second connecting portion 39 thereof is connected to the second sending passage 19, and the sensor unit 25 of the Raman spectrometer 11 is connected to the sensor unit connector 36. The culture supernatant liquid 15A, which is obtained from the culture vessel 13 in which the antibody-producing cell 16 is being cultured, is caused to flow in the flow passage 37 of the flow cell 10. The culture supernatant liquid 15A flowing through the flow passage 37 is irradiated with the excitation light EL via the sensor unit 25 and the optical system 63. The excitation light EL is condensed at the condensing position FP on the emission surface 70 of the transparent plate 59.

Raman scattered light is generated due to an interaction between the excitation light EL and the antibody 17 or the like contained in the culture supernatant liquid 15A. The Raman scattered light is taken into the sensor unit 25 by the optical system 63 and is output from the sensor unit 25 to the analyzer 26. The Raman scattered light is converted into the Raman spectral data 27 by the analyzer 26.

The emission surface 70 of the transparent plate 59, which forms the optical system 63, for the excitation light EL is in contact with the culture supernatant liquid 15A that flows through the flow passage 37. The emission surface 70 is a flat surface. As compared to the case of JP2020-511635A in which an emission surface 56 of a ball lens 46 having a positive optical power is in contact with culture supernatant liquid 15A, the distance d from the emission surface 70 for the excitation light EL to the condensing position FP can be shortened (reduced to 0 in this example). For this reason, it is possible to reduce a concern that the excitation light EL may be attenuated by the culture supernatant liquid 15A. Therefore, it is possible to reduce a concern that the amount of the excitation light EL required for obtaining the Raman spectral data 27 capable of withstanding analysis may not be obtained. As a result, an S/N ratio of the Raman spectral data 27 caused by the Raman scattered light generated due to an interaction between the excitation light EL and the antibody 17 or the like contained in the culture supernatant liquid 15A can be maintained at a high level.

The optical system 63 includes the ball lens 46 having a positive optical power and the transparent plate 59 of which the emission surface 70 for the excitation light EL in contact with the culture supernatant liquid 15A is a flat surface. For this reason, it is possible to realize a reduction in the condensation of the excitation light EL and a reduction in the attenuation of the excitation light EL with a simple configuration.

The ball lens 46 has a relatively high optical power, and the distance d between the first point P1 at which the emission surface 56 and the optical axis OA intersect with each other and the condensing position FP can be reduced. For this reason, the thickness Th of the transparent plate 59 in the direction of the optical axis OA can be reduced by that much. As a result, it is possible to contribute to a reduction in the size of the flow cell 10.

As shown in Fig. 4, the transparent plate 59 is a disk of which the emission surface 70 for the excitation light EL in contact with the culture supernatant liquid 15A and the incident surface 71 for the excitation light EL opposite to the emission surface 70 are parallel to each other. For this reason, the transparent plate 59 can be easily manufactured.

As shown in Fig. 6, the thickness Th of the transparent plate 59 in the direction of the optical axis OA is equal to the distance d between the first point P1 at which the emission surface 56 of the ball lens 46 and the optical axis OA intersect with each other and the condensing position FP, and the condensing position FP is positioned on the emission surface 70 of the transparent plate 59. For this reason, it is possible to reduce a concern that the excitation light EL may be attenuated by the culture supernatant liquid 15A to the utmost, and to significantly increase the amount of the excitation light EL required for obtaining the Raman spectral data 27 capable of withstanding analysis.

Further, as shown in Fig. 6, the thickness Th of the transparent plate 59 in the direction of the optical axis OA is 0.3 mm or more and 7.5 mm or less. In a case where the thickness Th is 0.3 mm or more, an effect of reducing a concern that the excitation light EL may be attenuated by the culture supernatant liquid 15A can be exhibited. In a case where the thickness Th is 7.5 mm or less, it is possible to contribute to a reduction in the size of the flow cell 10 and to obtain good handleability.

As shown in Fig. 2 and the like, the flow cell 10 comprises the sensor unit connector 36 to which the sensor unit 25 of the Raman spectrometer 11 outputting the Raman spectral data 27 is connected. The ball lens 46 is disposed at the distal end of the sensor unit connector 36. The sensor unit connector 36 can be attached to and detached from the main body 35. For this reason, the sensor unit connector 36 can be reused for a plurality of flow cells 10. Since it is not necessary to provide the sensor unit connector 36 in all the flow cells 10, it is possible to contribute to a reduction in the cost of the flow cell 10.

As shown in Fig. 6, the wall surface 57 forming the flow passage 37 is a smooth surface. For this reason, it is possible to reduce a concern that air bubbles hindering the flow of the culture supernatant liquid 15A may be generated. It is possible to ensure the stability of the flow of the culture supernatant liquid 15A.

As shown in Fig. 1, the turbidity of the culture supernatant liquid 15A is 250 NTU or more and 1000 NTU or less. In this case, since the attenuation of the excitation light EL caused by the culture supernatant liquid 15A is further increased, an effect obtained by making the emission surface 70 for the excitation light EL, which is in contact with the culture supernatant liquid 15A, as a flat surface can be further exhibited.

The Raman scattered light is likely to reflect information derived from a functional group of amino acid of protein. For this reason, in a case where the Raman spectral data 27 is used as physical property data as in this example, it is possible to derive a physical property value, such as the concentration of the antibody 17 which is protein, with high accuracy.

A biopharmaceutical including the antibody 17, which is a cell product, is called an antibody drug and is widely used not only for the treatment of chronic diseases, such as cancer, diabetes, and rheumatoid arthritis, but also for the treatment of rare diseases, such as hemophilia and a Crohn's disease. For this reason, according to this example in which the culture supernatant liquid 15A, which is obtained from the culture vessel 13 in which the antibody-producing cell 16 is being cultured and serves as a source of an antibody drug, is used as the fluid, it is possible to promote the development of the antibody drug that is widely used for the treatment of various diseases.

In this example, the culture supernatant liquid 15A, which is obtained from the culture vessel 13 in which culture is being performed, is used as the fluid. For this reason, it is possible to measure the Raman spectral data 27 while continuing to culture the antibody-producing cell 16 in the culture vessel 13.

The transparent plate 59 is disposed at the boundary portion 58 between the wall surface 57, which forms the flow passage 37, and the mounting portion 44 in this example, but the present disclosure is not limited thereto. The second holding portion 64 that holds the transparent plate 59 and the third holding portion 66 that holds the plate presser 61 may protrude from the boundary portion 58 toward a central portion of the flow passage 37, and the transparent plate 59 and the like may be disposed such that the emission surface 70 is positioned at the central portion of the flow passage 37.

### (Modification example 1)

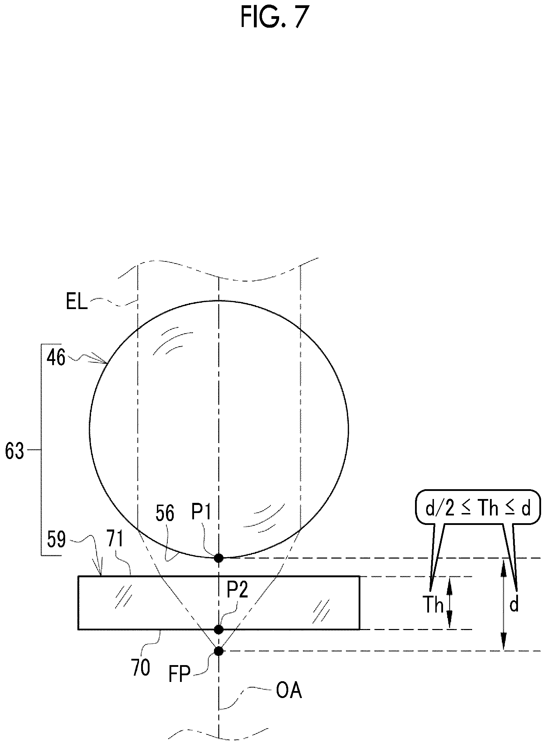

For example, as shown in Fig. 7, the emission surface 56 of the ball lens 46 and the incident surface 71 of the transparent plate 59 may not be in contact with each other. Further, the condensing position FP may not coincide with the second point P2 at which the emission surface 70 of the transparent plate 59 and the optical axis OA intersect with each other. That is, the condensing position FP may not be positioned on the emission surface 70 of the transparent plate 59.

However, in this case, the thickness Th of the transparent plate 59 in the direction of the optical axis OA is equal to or greater than a half of the distance d between the first point P1 at which the emission surface 56 of the ball lens 46 and the optical axis OA intersect with each other and the condensing position FP, and is equal to or smaller than the distance d (d/2 ≤ Th ≤ d). In a case where the thickness Th is equal to or greater than a half of the distance d, an effect of reducing a concern that the excitation light EL may be attenuated by the culture supernatant liquid 15A can be exhibited. In a case where the thickness Th is equal to or smaller than the distance d, the condensing position FP can be always set outside the transparent plate 59 (inside the flow passage 37).

### (Modification example 2)

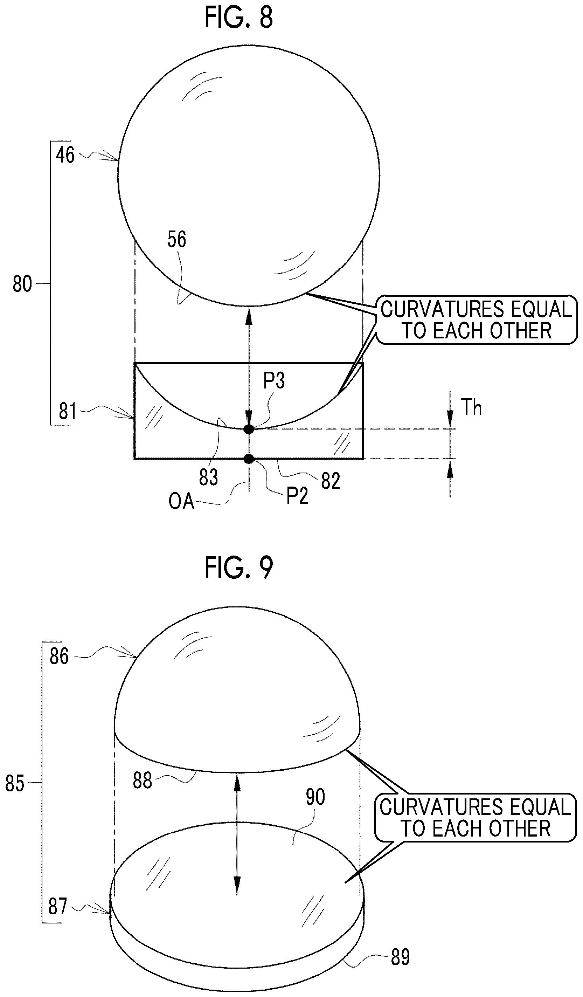

For example, an optical system 80 of Modification example 2 shown in Fig. 8 includes the ball lens 46 and an optical element 81. The optical element 81 includes an emission surface 82 for the excitation light EL that is a flat surface and is in contact with the culture supernatant liquid 15A, and an incident surface 83 that has a shape following the emission surface 56 of the ball lens 46. The curvature of the emission surface 56 of the ball lens 46 and the curvature of the incident surface 83 of the optical element 81 are equal to each other, and the emission surface 56 and the incident surface 83 are bonded to each other. Here, "bonding" may mean that the emission surface 56 and the incident surface 83 are fixed and bonded to each other with an adhesive or the like or the emission surface 56 and the incident surface 83 are held in a state where the surfaces are simply joined to each other without an adhesive or the like.

A thickness Th of the optical element 81 in the direction of the optical axis OA is a distance between a second point P2 at which the emission surface 82 of the optical element 81 and the optical axis OA intersect with each other and a third point P3 at which the incident surface 83 of the optical element 81 and the optical axis OA intersect with each other.

Since the curvature of the emission surface 56 and the curvature of the incident surface 83 are equal to each other, a gap does not occur between the emission surface 56 and the incident surface 83 that are bonded to each other. For this reason, it is possible to suppress the attenuation of the excitation light EL and the Raman scattered light caused by the gap between the emission surface 56 and the incident surface 83.

Here, "equal" in curvature refers to "equal" in a meaning including an error that is generally allowed in the technical field to which the technology of the present disclosure belongs and an error to such an extent not contrary to the spirit and scope of the technology of the present disclosure, in addition to completely equal. More specifically, "equal" in curvature means a case where a difference between the curvature of the emission surface 56 and the curvature of the incident surface 83 is within 10%. In a case where one having a smaller absolute value between the curvature of the emission surface 56 and the curvature of the incident surface 83 is denoted by m and the other having a larger absolute value therebetween is denoted by n, this relationship is represented by an expression of "0.9 × |n| ≤ Iml". The definition of "equal" in curvature is also applied to Figs. 9 to 12 to be described later. "Equal" in curvature preferably means a case where a difference between the curvature of the emission surface 56 and the curvature of the incident surface 83 is within 8%, more preferably means a case where the difference is within 5%, and still more preferably means a case where the difference is within 3%. The curvature can be measured with a device such as an ultra-high accuracy three-dimensional measuring instrument UA3P manufactured by Panasonic Production Engineering Co., Ltd. The curvature of the emission surface 56 can be derived from the number of Newton's rings appearing in a case where a Newton prototype and the emission surface 56 are superimposed.

As described above, the optical element is not limited to the disk-shaped transparent plate 59 including the emission surface 70 and the incident surface 71 parallel to each other, and may be the optical element 81 that includes the emission surface 82 which is a flat surface and the incident surface 83 having a shape following the emission surface 56 of the ball lens 46. Further, a lens to be combined with an optical element that includes an emission surface which is a flat surface and an incident surface having a shape following an emission surface of the lens, such as the optical element 81, is not limited to the exemplified ball lens 46 and may be a plano-convex lens, a biconvex lens, or the like.

### (Modification example 3)

For example, an optical system 85 of Modification example 3 shown in Fig. 9 includes a hemispherical lens 86 and a transparent plate 87. The hemispherical lens 86 is literally a lens having a hemispherical shape, and is made of, for example, quartz glass. The hemispherical lens 86 includes an emission surface 88 for the excitation light EL. The emission surface 88 is a flat surface. The hemispherical lens 86 is an example of "a lens having a positive optical power" according to the technology of the present disclosure.

The transparent plate 87 is a disk that includes an emission surface 89 and an incident surface 90 for the excitation light EL parallel to each other, like the transparent plate 59. The transparent plate 87 is an example of "an optical element" according to the technology of the present disclosure. The curvature of the emission surface 88 of the hemispherical lens 86 and the curvature of the incident surface 90 of the transparent plate 87 are equal to each other (0 in this case), and the emission surface 88 and the incident surface 90 are bonded to each other. Since the emission surface 88 is a flat surface in the hemispherical lens 86, the curvature of the incident surface 90 of the transparent plate 87 can be easily made to be equal to the curvature of the emission surface 88 as compared to the case of Fig. 8. Further, in a case where an emission surface of a lens and an incident surface of a transparent plate are flat surfaces even in the following examples, the curvatures of surfaces are 0.

### (Modification example 4)

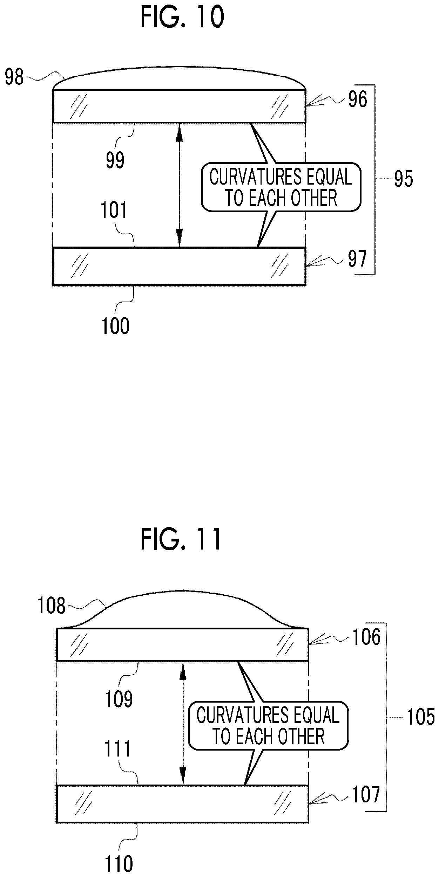

For example, an optical system 95 of Modification example 4 shown in Fig. 10 includes a plano-convex lens 96 and a transparent plate 97. The plano-convex lens 96 is a lens of which an incident surface 98 for the excitation light EL is a spherical convex surface and an emission surface 99 for the excitation light EL is a flat surface, and is made of, for example, quartz glass. The plano-convex lens 96 is an example of "a lens having a positive optical power" according to the technology of the present disclosure.

The transparent plate 97 is a disk that includes an emission surface 100 and an incident surface 101 for the excitation light EL parallel to each other, like the transparent plate 59 and the like. The transparent plate 97 is an example of "an optical element" according to the technology of the present disclosure. The curvature of the emission surface 99 of the plano-convex lens 96 and the curvature of the incident surface 101 of the transparent plate 97 are equal to each other (0 in this case), and the emission surface 99 and the incident surface 101 are bonded to each other. Even with the plano-convex lens 96, as in the case of the hemispherical lens 86, the curvature of the incident surface 101 of the transparent plate 97 can be easily made to be equal to the curvature of the emission surface 99 as compared to the case of Fig. 8. Further, since the plano-convex lens 96 is less expensive than the ball lens 46 and the hemispherical lens 86, it is possible to contribute to a reduction in the cost of the flow cell 10.

### (Modification example 5)

For example, an optical system 105 of Modification example 5 shown in Fig. 11 includes a plano-convex lens 106 and a transparent plate 107. The plano-convex lens 106 is a lens of which an incident surface 108 for the excitation light EL is an aspherical convex surface and an emission surface 109 for the excitation light EL is a flat surface, and is made of, for example, quartz glass or a resin. The plano-convex lens 106 is an example of "a lens having a positive optical power" according to the technology of the present disclosure.

The transparent plate 107 is a disk that includes an emission surface 110 and an incident surface 111 for the excitation light EL parallel to each other, like the transparent plate 59 and the like. The transparent plate 107 is an example of "an optical element" according to the technology of the present disclosure. The curvature of the emission surface 109 of the plano-convex lens 106 and the curvature of the incident surface 111 of the transparent plate 107 are equal to each other (0 in this case), and the emission surface 109 and the incident surface 111 are bonded to each other. Even with the plano-convex lens 106, as in the cases of the hemispherical lens 86 and the like, the curvature of the incident surface 111 of the transparent plate 107 can be easily made to be equal to the curvature of the emission surface 109 as compared to the case of Fig. 8. Further, since the incident surface 108 of the plano-convex lens 106 has an aspherical shape, spherical aberration can be suppressed as compared to the plano-convex lens 96 including the spherical incident surface 98.

### (Modification example 6)

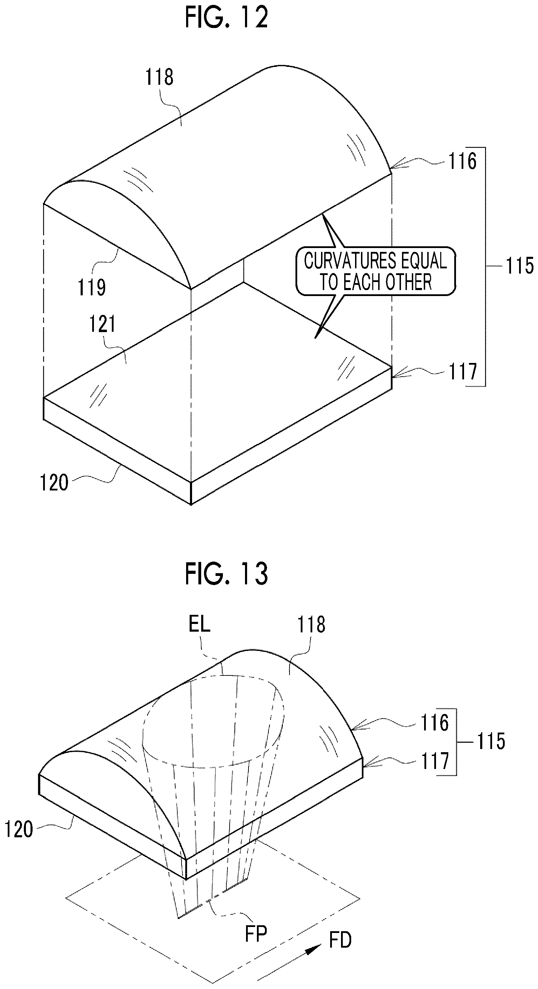

For example, an optical system 115 of Modification example 6 shown in Fig. 12 includes a cylindrical lens 116 and a transparent plate 117. The cylindrical lens 116 is a lens having a vertically split circular cylinder shape, and is made of, for example, quartz glass. The cylindrical lens 116 includes an incident surface 118 for the excitation light EL, which is a spherical convex surface, and an emission surface 119 for the excitation light EL. The emission surface 119 is a rectangular flat surface. The cylindrical lens 116 is an example of "a lens having a positive optical power" according to the technology of the present disclosure. The incident surface 118 of the cylindrical lens 116 may be an aspherical surface.

The transparent plate 117 is a rectangular plate that includes an emission surface 120 and an incident surface 121 for the excitation light EL parallel to each other. The transparent plate 117 is an example of "an optical element" according to the technology of the present disclosure. The curvature of the emission surface 119 of the cylindrical lens 116 and the curvature of the incident surface 121 of the transparent plate 117 are equal to each other (0 in this case), and the emission surface 119 and the incident surface 121 are bonded to each other. Even with the cylindrical lens 116, as in the cases of the hemispherical lens 86 and the like, the curvature of the incident surface 121 of the transparent plate 117 can be easily made to be equal to the curvature of the emission surface 119 as compared to the case of Fig. 8.

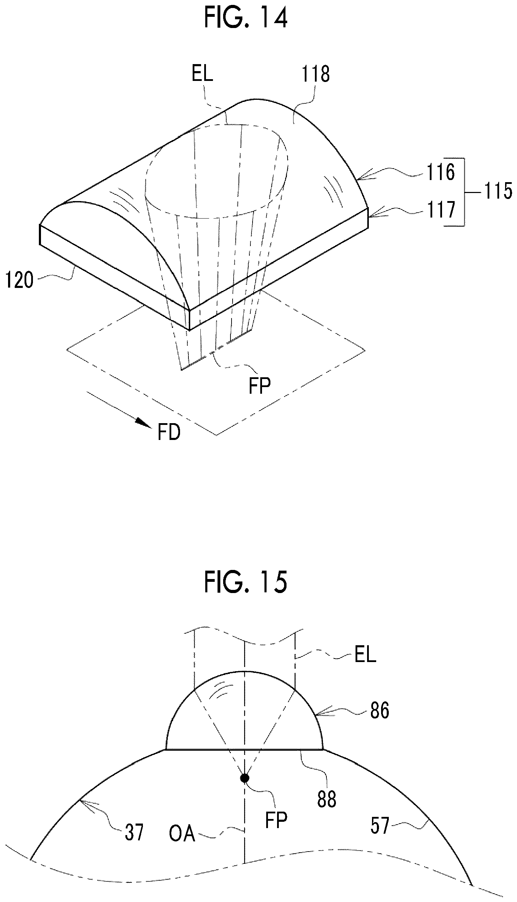

The ball lens 46, the hemispherical lens 86, and the plano-convex lenses 96 and 106 condense the excitation light EL in the form of a dot, but the cylindrical lens 116 condenses the excitation light EL in the form of a line as shown in, for example, Figs. 13 and 14. Fig. 13 shows a case where the excitation light EL is condensed in the form of a line parallel to the direction FD. On the other hand, Fig. 14 shows a case where the excitation light EL is condensed in the form of a line perpendicular to the direction FD.

In a case where the excitation light EL is condensed in the form of a line parallel to the direction FD as shown in Fig. 13, the temporal change of the culture supernatant liquid 15A flowing through the flow passage 37 can be reflected in the Raman spectral data 27. On the other hand, in a case where the excitation light EL is condensed in the form of a line perpendicular to the direction FD as shown in Fig. 14, the spatial change of the culture supernatant liquid 15A flowing through the flow passage 37 can be reflected in the Raman spectral data 27. In either case, the measurement stability of the Raman spectral data 27 can be improved as compared to a case where the excitation light EL is condensed in the form of a dot.

In Modification examples 2 to 6, the curvature of the emission surface of the lens and the curvature of the incident surface of the optical element are set to be equal to each other, and the emission surface of the lens and the incident surface of the optical element are bonded to each other. However, the present disclosure is not limited thereto. An aspect in which the curvature of the emission surface of the lens and the curvature of the incident surface of the optical element are set to be equal to each other but the emission surface of the lens and the incident surface of the optical element are not bonded to each other is also included in the technology of the present disclosure. "Not being bonded to each other" is synonymous with "not being in contact with each other" in a case where "the emission surface 56 of the ball lens 46 and the incident surface 71 of the transparent plate 59 may not be in contact with each other." is described in Modification example 1 shown in Fig. 7.

### [Second embodiment]

An optical in which a lens and an optical element are combined with each other, such as the optical system 63 including the ball lens 46 and the transparent plate 59, has been exemplified in the first embodiment, but the present disclosure is not limited thereto. For example, as shown in Fig. 15, an optical system may be formed of only the hemispherical lens 86. In this case, the emission surface 88 of the hemispherical lens 86 for the excitation light EL is in contact with the culture supernatant liquid 15A that flows through the flow passage 37. The emission surface 88 is an example of "an emission surface for measurement light in contact with fluid" and "a flat surface" according to the technology of the present disclosure.

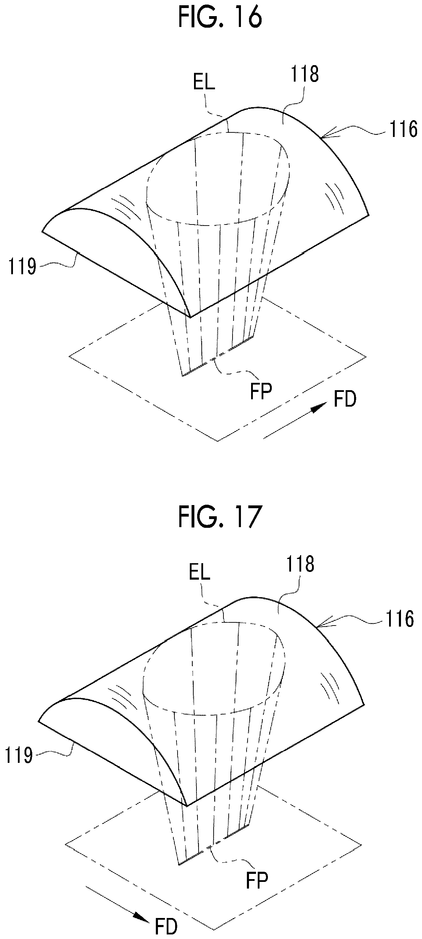

Further, for example, as shown in Figs. 16 and 17, an optical system may be formed of only the cylindrical lens 116. In this case, the emission surface 119 of the cylindrical lens 116 for the excitation light EL is in contact with the culture supernatant liquid 15A that flows through the flow passage 37. The emission surface 119 is an example of "an emission surface for measurement light in contact with fluid" and "a flat surface" according to the technology of the present disclosure. Furthermore, Fig. 16 shows a case where the excitation light EL is condensed in the form of a line parallel to the direction FD as in the case of Fig. 13. On the other hand, Fig. 17 shows a case where the excitation light EL is condensed in the form of a line perpendicular to the direction FD as in the case of Fig. 14.

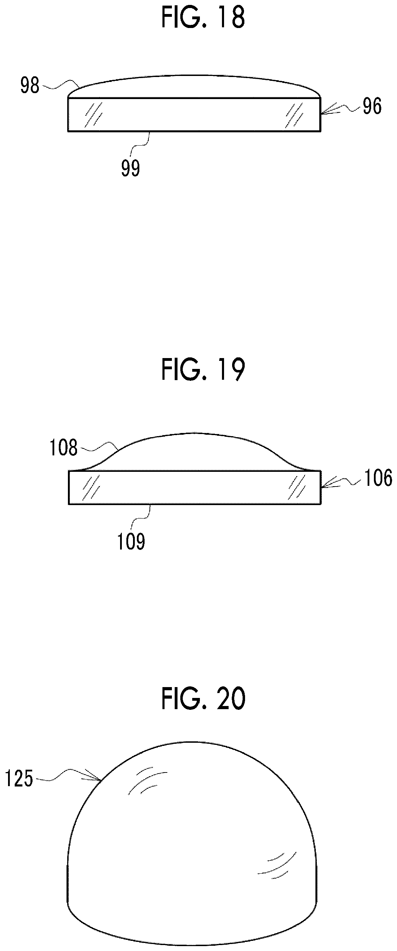

As described above, in the second embodiment, the optical system includes the lens which has a positive optical power and of which the emission surface for the excitation light EL in contact with the culture supernatant liquid 15A is a flat surface. For this reason, the optical system can be made to have a simple configuration as compared to the optical system of the first embodiment in which the lens and the optical element are combined with each other. As shown in Fig. 18, an optical system may be formed of only the plano-convex lens 96 shown in Fig. 10. Further, as shown in Fig. 19, an optical system may be formed of only the plano-convex lens 106 shown in Fig. 11.

Instead of forming an optical system with a lens having a positive optical power and an optical element that are formed separately, a lens having a positive optical power and an optical element may be integrally formed as one lens, such as an optical system 125 shown in Fig. 20 in which the hemispherical lens 86 and the transparent plate 87 shown in Fig. 9 are integrally formed as one lens.

### [Examples]

Hereinafter, Examples and Comparative examples of the technology of the present disclosure will be described.

In Examples, a phenylalanine solution was used as fluid used for measuring the Raman spectral data 27. L(-)-phenylalanine manufactured by FUJIFILM Wako Pure Chemical Corporation was dissolved in water, which was a solvent, to make a concentration of 10 g/L, so that the phenylalanine solution was produced. Then, a powder culture medium was put into the produced phenylalanine solution to change the turbidity of the phenylalanine solution. Phenylalanine is one of typical amino acids contained in the cell culture solution, and was employed since a peak in the Raman spectral data 27 was relatively easily discriminated. For example, CD OptiCHO^{™} AGT^{™} Medium manufactured by Thermo Fisher Scientific, Inc. can be used as the powder culture medium. Turbidity was measured using a digital turbidity meter TU-2016 (manufactured by Sato Keiryoki Mfg. Co., Ltd.).

The hemispherical lens 86 (Examples 1 to 7) or the ball lens 46 (Examples 8 to 10 and Comparative examples 1 to 3) was used as a lens. The diameters of the hemispherical lens 86 and the ball lens 46 are 8 mm, and the hemispherical lens 86 and the ball lens 46 are made of quartz glass (S-BSL).

The produced phenylalanine solution was injected into the flow passage 37 using a syringe. The phenylalanine solution injected into the flow passage 37 was irradiated with the excitation light EL emitted from the Raman spectrometer 11. Laser light was used as the excitation light EL, an output of the laser light was set to 500 mW, a central wavelength thereof was set to 785 nm, an irradiation time thereof was set to 1 second, and the number of times of integration thereof was set to 10. Then, the obtained raw Raman spectral data 27 was subjected to baseline correction processing, and a difference between the raw Raman spectral data 27 and the Raman spectral data 27 of water, which was the solvent subjected to the baseline correction processing in the same manner, was used as final Raman spectral data 27 used for evaluating an S/N ratio.

The maximum intensity of intensities at a wave number of 1000 cm⁻¹ to 1011 cm⁻¹ belonging to phenylalanine in the Raman spectral data 27 was used as a signal value used for calculating an S/N ratio. Further, in the Raman spectral data 27, a standard deviation of intensities at a wave number of 695 cm⁻¹ to 721 cm⁻¹ which are not derived from other amino acids including phenylalanine was used as a noise value used for calculating an S/N ratio.

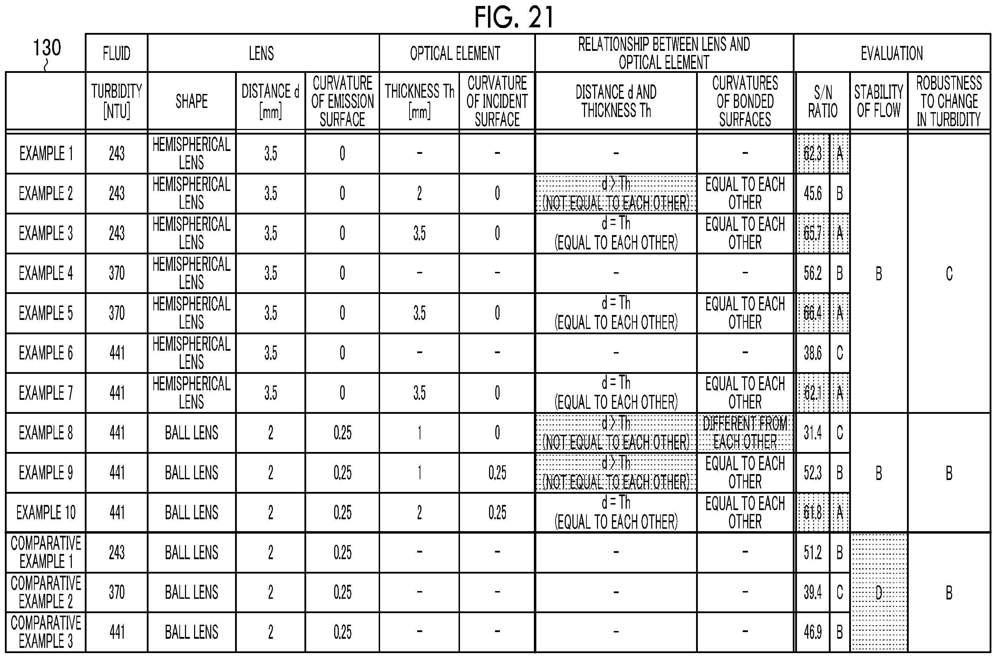

Turbidity was changed or the thickness Th of the optical element (transparent plate) in the direction of the optical axis OA was changed to evaluate each example. In addition to the above-described S/N ratio, the stability of the flow of the fluid and robustness to a change in turbidity were also evaluated. Fig. 21 shows Table 130 in which items of conditions and evaluation in Examples and Comparative examples are summarized. With regard to evaluation, "A" is excellent, "B" is good, "C" is acceptable, and "D" is unacceptable.

Example 1 is an example in which the turbidity of the phenylalanine solution is 243 NTU, the lens is the hemispherical lens 86, the distance d is 3.5 mm, the curvature of the emission surface 88 is 0, and the optical element (transparent plate 87) is not provided. That is, Example 1 is the aspect shown in Fig. 15. The S/N ratio in Example 1 was 62.3 and was evaluated as "A". The reason why the S/N ratio was evaluated as "A" was that the emission surface 88 of the hemispherical lens 86 for the excitation light EL in contact with the phenylalanine solution was a flat surface.

Example 2 is an example in which the transparent plate 87 of which the thickness Th is 2 mm and the curvature of the incident surface 90 is 0 is bonded to the emission surface 88 of the hemispherical lens 86 in Example 1. Example 2 is an example in which the curvature of the emission surface 88 of the hemispherical lens 86 and the curvature of the incident surface 90 of the transparent plate 87 are equal to each other. That is, Example 2 is the aspect shown in Fig. 9. Further, Example 2 is an example in which the thickness Th is smaller than the distance d and the distance d and the thickness Th are not equal to each other. The S/N ratio in Example 2 was 45.6 and was evaluated as "B". The reason why the S/N ratio was evaluated as "B", which was worse than that in Example 1, was that the distance d and the thickness Th were not equal to each other.

Example 3 is an example in which the transparent plate 87 of which the thickness Th is 3.5 mm and the curvature of the incident surface 90 is 0 is bonded to the emission surface 88 of the hemispherical lens 86 in Example 1. Example 3 is also an example in which the curvature of the emission surface 88 of the hemispherical lens 86 and the curvature of the incident surface 90 of the transparent plate 87 are equal to each other as in Example 2. That is, Example 3 is also the aspect shown in Fig. 9. However, Example 3 is an example in which the distance d and the thickness Th are equal to each other. The S/N ratio in Example 3 was 65.7 and was evaluated as "A". The reason why the S/N ratio was evaluated as "A", which was improved as compared to Example 2, was that the transparent plate 87 in which the distance d and the thickness Th were equal to each other was provided.

Example 4 is the same as Example 1 except that the turbidity of the phenylalanine solution is set to 370 NTU. The S/N ratio in Example 4 was 56.2 and was evaluated as "B". The reason why the S/N ratio was evaluated as "B", which was worse than that in Example 1, was that the turbidity of the phenylalanine solution was increased.

Example 5 is the same as Example 3 except that the turbidity of the phenylalanine solution is set to 370 NTU. The S/N ratio in Example 5 was 66.4 and was evaluated as "A". The reason why the S/N ratio was evaluated as "A", which was improved as compared to Example 4, was that the transparent plate 87 in which the distance d and the thickness Th were equal to each other was provided.

Example 6 is the same as Examples 1 and 4 except that the turbidity of the phenylalanine solution is set to 441 NTU. The S/N ratio in Example 6 was 38.6 and was evaluated as "C". The reason why the S/N ratio was evaluated as "C", which was worse than those in Examples 1 and 4, was that the turbidity of the phenylalanine solution was increased.

Example 7 is the same as Examples 3 and 5 except that the turbidity of the phenylalanine solution is set to 441 NTU. The S/N ratio in Example 7 was 62.1 and was evaluated as "A". The reason why the S/N ratio was evaluated as "A", which was improved as compared to Example 6, was that the transparent plate 87 in which the distance d and the thickness Th were equal to each other was provided.

In Examples 1 to 7 in which only the hemispherical lens 86 was used or the hemispherical lens 86 and the transparent plate 87 were used, the stability of the flow was evaluated as "B" and robustness to a change in turbidity was evaluated as "C".

Example 8 is an example in which the turbidity of the phenylalanine solution is 441 NTU, the lens is the ball lens 46, the distance d is 2 mm, the curvature of the emission surface 56 is 0.25, and the transparent plate 59 of which the thickness Th is 1 mm and the curvature of the incident surface 71 is 0 is provided. Example 8 is an example in which the thickness Th is smaller than the distance d and the distance d and the thickness Th are not equal to each other. Further, Example 8 is an example in which the curvature of the emission surface 56 of the ball lens 46 and the curvature of the incident surface 71 of the transparent plate 59 are different from each other. The S/N ratio in Example 8 was 31.4 and was evaluated as "C". The reason why the S/N ratio was evaluated as "C" was that the distance d and the thickness Th were not equal to each other and the curvature of the emission surface 56 of the ball lens 46 and the curvature of the incident surface 71 of the transparent plate 59 were different from each other.

Example 9 is an example in which the optical element 81 of which the thickness Th is 1 mm and the curvature of the incident surface 83 is 0.25 is bonded to the emission surface 56 of the ball lens 46 instead of the transparent plate 59 of Example 8 of which the thickness Th is 1 mm and the curvature of the incident surface 71 is 0. That is, Example 9 is the aspect shown in Fig. 8. Example 9 is the same as Example 8 in that the thickness Th is smaller than the distance d and the distance d and the thickness Th are not equal to each other, but Example 9 is different from Example 8 in that the curvature of the emission surface 56 of the ball lens 46 and the curvature of the incident surface 83 of the optical element 81 are equal to each other. The S/N ratio in Example 9 was 52.3 and was evaluated as "B". The reason why the S/N ratio was evaluated as "B", which was improved as compared to Example 8, was that the curvature of the emission surface 56 of the ball lens 46 and the curvature of the incident surface 83 of the optical element 81 were equal to each other.

Example 10 is an example in which the optical element 81 of which the thickness Th is 2 mm and the curvature of the incident surface 83 is 0.25 is bonded to the emission surface 56 of the ball lens 46. Example 10 is also an example in which the curvature of the emission surface 56 of the ball lens 46 and the curvature of the incident surface 83 of the optical element 81 are equal to each other as in Example 9. That is, Example 10 is also the aspect shown in Fig. 8. However, Example 10 is an example in which the distance d and the thickness Th are equal to each other. The S/N ratio in Example 10 was 61.8 and was evaluated as "A". The reason why the S/N ratio was evaluated as "A", which was improved as compared to Example 9, was that the optical element 81 in which the distance d and the thickness Th were equal to each other was provided.

In Examples 8 to 10 in which the ball lens 46 and the transparent plate 59 or 81 were used, the stability of the flow was evaluated as "B" and robustness to a change in turbidity was evaluated as "B". The reason why robustness to a change in turbidity was evaluated as "B", which was improved as compared to Examples 1 to 7 in which the hemispherical lens 86 was used, was that the ball lens 46 in which the distance d was short as compared to the hemispherical lens 86 was used.

Comparative example 1 is an example in which the turbidity of the phenylalanine solution is 243 NTU, the lens is the ball lens 46, the distance d is 2 mm, the curvature of the emission surface 56 is 0.25, and the optical element (transparent plate 59 or 81) is not provided. That is, Comparative example 1 is the aspect described in JP2020-511635A. The S/N ratio in Comparative example 1 was 51.2 and was evaluated as "B".

Comparative example 2 is the same as Comparative example 1 except that the turbidity of the phenylalanine solution is set to 370 NTU. The S/N ratio in Comparative example 2 was 39.4 and was evaluated as "C". The reason why the S/N ratio was evaluated as "C", which was worse than that in Comparative example 1, was that the turbidity of the phenylalanine solution was increased.

Comparative example 3 is the same as Comparative example 1 except that the turbidity of the phenylalanine solution is set to 441 NTU. The S/N ratio in Comparative example 3 was 46.9 and was evaluated as "B". A cause of the evaluation of the S/N ratio as "B", which is improved as compared to Comparative example 2, is unclear.

In Comparative examples 1 to 3 in which only the ball lens 46 was used, the stability of the flow was evaluated as "D" and robustness to a change in turbidity was evaluated as "B". The reason why the flow stability was evaluated as "D" was that a surface in contact with the flow passage 37 was the emission surface 56 of the ball lens 46 and was not a flat surface.

The S/N ratios in Examples 1 to 10 to which the technology of the present disclosure is applied are all within the range of "A" to "C". Therefore, an effect of the technology of the present disclosure capable of reducing a concern that the amount of the excitation light EL required for obtaining the Raman spectral data 27 capable of withstanding analysis may not be obtained was verified.

In Examples 1 to 3 and Comparative example 1 having in common that the turbidity of the phenylalanine solution is 243 NTU, the S/N ratios in Examples 1 and 3 are higher than the S/N ratio in Comparative example 1. Further, in Examples 4 and 5 and Comparative example 2 having in common that the turbidity of the phenylalanine solution is 370 NTU, the S/N ratios in Examples 4 and 5 are higher than the S/N ratio in Comparative example 2. Furthermore, in Examples 6 to 10 and Comparative example 3 having in common that the turbidity of the phenylalanine solution is 441 NTU, the S/N ratios in Examples 7, 9, and 10 are higher than the S/N ratio in Comparative example 3. Therefore, an effect capable of more obtaining the amount of the excitation light EL required for obtaining the Raman spectral data 27 capable of withstanding analysis than that in the related art was verified.

The lens is not limited to the ball lens 46, the hemispherical lens 86, the plano-convex lenses 96 and 106, or the cylindrical lens 116 having been exemplified. Both a biconvex lens in which an incident surface and an emission surface of the excitation light EL are convex surfaces and a fly's eye lens in which a plurality of lenses are two-dimensionally arranged may be used.

The sensor unit connector 36 may be mounted on the main body 35 not to be attachable and detachable. The first connecting portion 38 and the second connecting portion 39 may be disposed on the lower side of the main body 35, and the flow passage 37 may have a U-shape. The shape of the main body 35 is not limited to a cylindrical shape, and may be a shape of a polygonal prism. The cross-sectional shape of the flow passage 37 is also not limited to a circular shape, and may be an elliptical shape or a rectangular shape. Further, the main body 35 and the like may be made of a composite material such as a polyolefin-based resin or a carbon fiber reinforced resin.

A substance from which the Raman spectral data 27 is to be measured is not limited to the antibody 17 and the like. The substance from which the Raman spectral data 27 is to be measured may be protein, peptide, nucleic acid (DNA or ribonucleic acid (RNA)), lipid, a virus, a virus subunit, a virus-like particle, and the like other than the antibody 17.

The cell product is not limited to the antibody 17 and the like. Examples of the cell product include cytokine (interferon, interleukin, or the like), hormone (insulin, glucagon, follicle-stimulating hormone, erythropoietin, or the like), a growth factor (insulin-like growth factor (IGF)-1, basic fibroblast growth factor (bFGF), or the like), a blood coagulation factor (a seventh factor, an eighth factor, a ninth factor, or the like), an enzyme (a lysosomal enzyme, a deoxyribonucleic acid (DNA) degrading enzyme, or the like), a fragment crystallizable (Fc) fusion protein, a receptor, albumin, and a protein vaccine. Further, examples of the antibody 17 include a bispecific antibody, an antibody-drug conjugate, a low-molecular-weight antibody, and a sugar-chain-modified antibody.

The physical property data is not limited to the Raman spectral data 27. The physical property data may be infrared absorption spectral data, nuclear magnetic resonance spectral data, ultraviolet visible absorption spectroscopy (UV-Vis) spectral data, or fluorescence spectral data. For this reason, the optical analysis apparatus is also not limited to the Raman spectrometer 11.

The fluid is not limited to the culture supernatant liquid 15A. The fluid may be the cell culture solution 15 from which cells are not yet removed by the cell-removing filter 14. The fluid may be a cell culture solution (so-called culture medium) that does not contain cell products and is not yet supplied to the culture vessel 13. The fluid may be a purified solution that is obtained in a case where the culture supernatant liquid 15A is purified with a chromatography device in the purification unit. The fluid is not limited to the cell culture solution 15, and may be, for example, river water collected to investigate water pollution. The fluid may be a raw material (for example, polystyryllithium, a methanol aqueous solution, and the like) and/or a product (for example, a monomer or a polymer such as polystyrene) in a case where a product, such as a monomer or a polymer (for example, polystyrene or the like), is continuously produced by flow synthesis. Further, the fluid is not limited to liquid and may be gas.

It is possible to understand technologies described in the following supplementary claims from the above description.

### [Supplementary claim 1]

A flow cell comprising:
a main body that includes a flow passage through which fluid containing a substance from which physical property data is to be measured flows; and
an optical system which is disposed on a part of a wall surface forming the flow passage and condenses measurement light for the physical property data and of which an emission surface for the measurement light in contact with the fluid is a flat surface.

### [Supplementary claim 2]

The flow cell according to supplementary claim 1,
wherein the optical system includes a lens that has a positive optical power, and an optical element of which an emission surface for the measurement light in contact with the fluid is a flat surface.

### [Supplementary claim 3]

The flow cell according to supplementary claim 2,
wherein the lens is any one of a ball lens, a hemispherical lens, or a cylindrical lens.

### [Supplementary claim 4]

The flow cell according to supplementary claim 3,
wherein, in a case where the lens is the cylindrical lens, the cylindrical lens condenses the measurement light in a form of a line parallel or perpendicular to a direction in which the fluid flows.

### [Supplementary claim 5]

The flow cell according to any one of supplementary claims 2 to 4,
wherein the optical element is a transparent plate of which an emission surface for the measurement light in contact with the fluid and an incident surface for the measurement light opposite to the emission surface for the measurement light in contact with the fluid are parallel to each other.

### [Supplementary claim 6]

The flow cell according to any one of supplementary claims 2 to 5,
wherein a thickness of the optical element in a direction of an optical axis is equal to or smaller than a distance between a point at which an emission surface of the lens and the optical axis intersect with each other and a condensing position of the measurement light.

### [Supplementary claim 7]

The flow cell according to supplementary claim 6,
wherein the thickness is equal to or greater than a half of the distance.

### [Supplementary claim 8]

The flow cell according to supplementary claim 6 or 7,
wherein the thickness is equal to the distance, and
the condensing position is positioned on the emission surface of the optical element.

### [Supplementary claim 9]

The flow cell according to any one of supplementary claims 6 to 8,
wherein the thickness is 0.3 mm or more and 7.5 mm or less.

### [Supplementary claim 10]

The flow cell according to any one of supplementary claims 2 to 9,
wherein a curvature of an emission surface of the lens and a curvature of an incident surface of the optical element are equal to each other.

### [Supplementary claim 11]

The flow cell according to supplementary claim 10,
wherein the emission surface of the lens and the incident surface of the optical element are bonded to each other.

### [Supplementary claim 12]

The flow cell according to any one of supplementary claims 2 to 11,
wherein a connector, to which an optical analysis apparatus outputting the physical property data is connected and in which the lens is disposed at a distal end, is attachably and detachably provided on the main body.

### [Supplementary claim 13]

The flow cell according to supplementary claim 1,
wherein the optical system includes a lens which has a positive optical power and of which an emission surface for the measurement light in contact with the fluid is a flat surface.

### [Supplementary claim 14]

The flow cell according to supplementary claim 13,
wherein the lens is any one of a hemispherical lens or a cylindrical lens.

### [Supplementary claim 15]

The flow cell according to claim 14,
wherein, in a case where the lens is the cylindrical lens, the cylindrical lens condenses the measurement light in a form of a line parallel or perpendicular to a direction in which the fluid flows.

### [Supplementary claim 16]

The flow cell according to any one of supplementary claims 1 to 15,
wherein the wall surface is a smooth surface.

### [Supplementary claim 17]

The flow cell according to any one of supplementary claims 1 to 16,
wherein a turbidity of the fluid is 250 NTU or more and 1000 NTU or less.

### [Supplementary claim 18]

The flow cell according to any one of supplementary claims 1 to 17,
wherein the physical property data is Raman spectral data.

### [Supplementary claim 19]

A measuring method of measuring the physical property data using the flow cell according to any one of supplementary claims 1 to 18.

### [Supplementary claim 20]

The measuring method according to supplementary claim 19,
wherein the fluid is a cell culture solution.

### [Supplementary claim 21]

The measuring method according to supplementary claim 20,
wherein the cell culture solution contains a cell product as the substance.

### [Supplementary claim 22]

The measuring method according to supplementary claim 20 or 21,
wherein the cell culture solution is obtained from a culture vessel in which culture is being performed.

### [Supplementary claim 23]

The measuring method according to any one of supplementary claims 20 to 22,
wherein the cell culture solution is a solution from which cells have been removed.

In the technology of the present disclosure, the above-described various embodiments and/or various modification examples may be combined with each other as appropriate. Further, it is natural that the present disclosure is not limited to each embodiment described above and may employ various configurations without departing from the scope.

The described contents and illustrated contents shown above are detailed descriptions of the parts related to the technology of the present disclosure, and are merely an example of the technology of the present disclosure. For example, the description of the configuration, functions, actions, and effects having been described above is the description of examples of the configuration, functions, actions, and effects of the portions according to the technology of the present disclosure. Accordingly, it goes without saying that unnecessary portions may be deleted or new elements may be added or replaced in the description contents and shown contents described above without departing from the scope of the technology of the present disclosure. Further, in order to avoid complications and facilitate understanding of the parts related to the technology of the present disclosure, descriptions of common general knowledge and the like that do not require special descriptions for enabling the implementation of the technology of the present disclosure are omitted, in the described contents and illustrated contents shown above.

In the present specification, the term "A and/or B" is synonymous with the term "at least one of A or B". That is, "A and/or B" may mean only A, may mean only B, or may mean a combination of A and B. In addition, in the present specification, the same approach as "A and/or B" is applied to a case where three or more matters are represented by connecting the matters with "and/or".

All documents, patent applications, and technical standards described in the present specification are incorporated in the present specification by reference to the same extent as in a case where each of the documents, patent applications, technical standards are specifically and individually indicated to be incorporated by reference.

## Claims

1. A flow cell comprising:
a main body that includes a flow passage through which fluid containing a substance from which physical property data is to be measured flows; and
an optical system which is disposed on a part of a wall surface forming the flow passage and condenses measurement light for the physical property data and of which an emission surface for the measurement light in contact with the fluid is a flat surface.

2. The flow cell according to claim 1,
wherein the optical system includes a lens that has a positive optical power, and an optical element of which an emission surface for the measurement light in contact with the fluid is a flat surface.

3. The flow cell according to claim 2,
wherein the lens is any one of a ball lens, a hemispherical lens, or a cylindrical lens.

4. The flow cell according to claim 3,
wherein, in a case where the lens is the cylindrical lens, the cylindrical lens condenses the measurement light in a form of a line parallel or perpendicular to a direction in which the fluid flows.

5. The flow cell according to claim 2,
wherein the optical element is a transparent plate of which an emission surface for the measurement light in contact with the fluid and an incident surface for the measurement light opposite to the emission surface for the measurement light in contact with the fluid are parallel to each other.

6. The flow cell according to claim 2,
wherein a thickness of the optical element in a direction of an optical axis is equal to or smaller than a distance between a point at which an emission surface of the lens and the optical axis intersect with each other and a condensing position of the measurement light.

7. The flow cell according to claim 6,
wherein the thickness is equal to or greater than a half of the distance.

8. The flow cell according to claim 6,
wherein the thickness is equal to the distance, and
the condensing position is positioned on the emission surface of the optical element.

9. The flow cell according to claim 6,
wherein the thickness is 0.3 mm or more and 7.5 mm or less.

10. The flow cell according to claim 2,
wherein a curvature of an emission surface of the lens and a curvature of an incident surface of the optical element are equal to each other.

11. The flow cell according to claim 10,
wherein the emission surface of the lens and the incident surface of the optical element are bonded to each other.

12. The flow cell according to claim 2,
wherein a connector, to which an optical analysis apparatus outputting the physical property data is connected and in which the lens is disposed at a distal end, is attachably and detachably provided on the main body.

13. The flow cell according to claim 1,
wherein the optical system includes a lens which has a positive optical power and of which an emission surface for the measurement light in contact with the fluid is a flat surface.

14. The flow cell according to claim 13,
wherein the lens is any one of a hemispherical lens or a cylindrical lens.

15. The flow cell according to claim 14,
wherein, in a case where the lens is the cylindrical lens, the cylindrical lens condenses the measurement light in a form of a line parallel or perpendicular to a direction in which the fluid flows.

16. The flow cell according to claim 1,
wherein the wall surface is a smooth surface.

17. The flow cell according to claim 1,
wherein a turbidity of the fluid is 250 NTU or more and 1000 NTU or less.

18. The flow cell according to claim 1,
wherein the physical property data is Raman spectral data.

19. A measuring method of measuring the physical property data using the flow cell according to claim 1.

20. The measuring method according to claim 19,
wherein the fluid is a cell culture solution.

21. The measuring method according to claim 20,
wherein the cell culture solution contains a cell product as the substance.

22. The measuring method according to claim 20,
wherein the cell culture solution is obtained from a culture vessel in which culture is being performed.

23. The measuring method according to claim 20,
wherein the cell culture solution is a solution from which cells have been removed.
